# EUROPEAN PATENT APPLICATION

(11) **EP 2 098 244 A1**
(43) Date of publication of application: **09.09.2009**
(21) Application number: 08102281.6
(22) Date of filing: 04.03.2008
(51) Int. Cl.: A61K 38/18

(54) **Pharmaceutical composition for the treatment of myocardial infarction**

(71) Applicant: Medizinische Hochschule Hannover, 30625 Hannover (DE)
(72) Inventor: Wollert, Kai C., Prof. Dr., 30559 Hannover (DE); Drexler, Helmut, Prof. Dr., 30559 Hannover (DE); Korf-Klingebiel, Mortimer, Dr., 37115 Duderstadt (DE)
(74) Representative: Taruttis, Stefan Georg

(57) **Abstract**

The present invention provides fibroblast growth factor-9 (FGF9) and Dickkopf homologue 1 (DKK1) singly and in combination for use in the treatment of heart diseases involving reperfusion damage and/or ischemic damage. As it has been found that FGF9 has a pronounced protective effect on the myocardium under ischemic and/or reperfusion conditions, FGF9 is provided for use in the production of a pharmaceutical composition for the treatment of ischemic conditions for the prevention of necrosis and/or apoptosis of cardiomyocytes.

## Description

The present invention relates to a pharmaceutical composition suitable for the medical treatment of heart diseases involving perfusion damage and/or ischemic damage as occurring in myocardial infarction, angina, cardiac insufficiency including chronic heart failure and congestive heart failure, arterial obstructive disease as well as generally heart diseases that would benefit from an angiogenic effect and/or from a protective effect against induced apoptosis or necrosis on cardiomyocytes.

### State of the art

CA 2 390 285 A1 describes the cardioprotective properties of fibroblast growth factor-2 (FGF2) and of a mutant FGF-2 having reduced mitogenic properties towards a non-ischemic as well as ischemic myocard.

WO 2007/136225 A1 describes that Dickkopf-1 protein (DKK1) inhibits angiogenesis in mammals and, accordingly, the pharmaceutical use of DKK1 against diseases caused by angiogenesis.

WO 02/066509 A2 discloses the use of antagonists to Dickkopf-1 protein, e.g. of an antibody binding to DKK1, e.g. for the treatment of non-insulin dependent diabetes mellitus. Further, the medical use of DKK1 in the treatment of obesity or hypoinsulinemia is described.

Lavine et al. (Genes and Development 1651 - 1666, 2006) quote that among other FGFs FGF9 is expressed in the embryonic heart during coronary formation. Lavine et al. conclude that hedgehog is the critical signal for coronary development in the embryonic murine heart as hedgehog controls the expression of multiple proangiogenic factors including VEGF-A, Vegf-B, Vegf-C and Ang2.

When investigating the expression of proteins in myocardium before and following explantation of a left ventricular assist device, Hall et al. (Physiol. Genomics 17: 283 - 291, 2004) have found 22 genes that were downregulated and 85 genes that were upregulated in response to mechanical unloading, including neurophilin, FGF9, Sprouty1, stromal-derived factor 1 and endomucin. The analysis of the data obtained according to Hall et al. indicates that BNP gene expression plays a central role in the heart response to mechanical unloading. Further, FOX 03A and FOX 01A are discussed in connection with forkhead, and angiotensin.

For the treatment of the infarcted heart, a physical standard therapy is to impart dilatation to the closed vessel, followed by integration of a stent to keep the vessel open.

### Objects of the invention

It is an object of the present invention to provide a pharmaceutical composition for the medical treatment of heart diseases that involve the occurrence of reperfusion damage and/or of ischemic damage, especially to cardiac tissue, e.g. to the myocardium, especially to cardiomyocytes. A preferred indication for administration of the pharmaceutical composition is myocardial infarction, angina, chronic heart failure, peripheral arterial obstructive disease, and vessel occlusion.

### General description of the invention

The present invention attains the above objects as it has been found that fibroblast growth factor-9 (FGF9) and Dickkopf homologue 1 (DKK1) singly and in combination have a pronounced angiogenic effect, making both factors suitable for their use in the treatment of heart diseases involving reperfusion damage and/or ischemic damage. Further, it has been found that FGF9 has a pronounced protective effect on the myocardium under ischemic and/or reperfusion conditions, and the medical administration of FGF9 to a heart being exposed to conditions normally inducing reperfusion damage and/or ischemic damage effectively reduces cell death, e.g. apoptosis and necrosis. Accordingly, FGF9 is suitable for use in the production of a pharmaceutical composition for the treatment of ischemic conditions for the prevention of necrosis and/or apoptosis of cardiomyocytes.

Further, the medical effects obtained by administration of FGF9 and/or DKK1 could be found when using nucleic acids encoding FGF9 and/or DKK1 instead of the protein. Preferably, the nucleic acid encoding FGF9 and/or DKK1 is contained in a eucaryotic expression cassette, e.g. comprised in a transduction vector, e.g. a lentiviral, retroviral or gamma-retroviral vector.

FGF9 and DKK1 preferably is the natural human protein, having the amino acid sequence according to Seq ID No. 1 for FGF9, and Seq ID No. 2 for DKK1, or functional derivatives or mutants thereof.

Functional mutants of FGF9 and DKK1 can be identified by formally exchanging at least one amino acid in the natural amino acid sequence, e.g. in Seq ID No. 1 or Seq ID No. 2. Functional derivatives of FGF9 and DKK1 can be obtained by formally deleting at least one amino acid from the amino acid sequence of FGF9 and DKK1. In order to assess the functionality, i.e. the medical effect of mutants and derivatives of FGF9 and DKK1, respectively, the analytical assays described herein for testing the activity of FGF9 and DKK1 in *in vitro* and/or *in vivo* can be employed.

For production of FGF9 and DKK1, it is preferred to express FGF9, DKK1 or functional derivatives and mutants thereof from a nucleic acid comprised in a micro-organism, with the nucleic acid encoding the amino acid sequence of the expression product.

For administration of FGF9 and/or of DKK1 to a patient for medical purposes, FGF9, DKK1 as well as functional derivatives and mutants thereof can be administered in the form of protein formulated in a suitable pharmaceutical composition, e.g. for injection, preferably for arterial or intramuscular injection. In the alternative or in addition to the formulation of FGF9 or DKK1 as protein in a pharmaceutical composition, FGF9, DKK1 and functional derivatives and mutants thereof can be administered to a patient in the form of a nucleic acid sequence encoding FGF9, DKK1 and/or functional mutants and derivatives thereof. A nucleic acid sequence encoding FGF9 and/or DKK1, functional derivatives and mutants thereof can be provided in the form of an expression cassette containing a eucaryotic promoter sequence in functional arrangement to the coding sequence, e.g. in 5' thereto, preferably with a polyadenylation site and/or terminator site arranged to the 3'-end of the coding sequence. Further, a nucleic acid sequence, preferably arranged between functional elements constituting a eucaryotic expression cassette, can be contained in a viral vector e.g. a retroviral or lentiviral vector, preferably a gamma-retroviral vector, suitable for pseudo-transduction of mammalian cells, especially of cardiomyocytes.

The present invention is based on the observation that during treatment of patients having suffered a myocardial infarction who receive treatment involving the administration of autologous bone marrow cells, a large array of factors secreted by the bone marrow cells could be identified that in contrast could be found in circulating blood only at significantly lower titers.

Detailed analysis of the array of factors lead to the identification of FGF9 protein as having anti-apoptotic and anti-necrotic effects on cardiomyocytes, which effect was demonstrated in simulated ischemia as well as under reperfusion conditions in neonatal ventricular cardiomyocytes. The protective effect of FGF9 on myocardium against conditions inducing apoptosis or necrosis was generally comparable to the cardioprotective effect of growth differentiation factor 15 (GDF-15).

Further, it could be shown that FGF9 has an angiogenic effect on endothelial cells in an *in vitro* assay. In detail, FGF9 could be shown to induce angiogenesis in cultivated endothelial cells and to exert protective effects against induced apoptotic or necrotic cell damage in cultivated cardiomyocytes as well as in the infarcted heart.

Further, DKK1 is identified to have angiogenic effects on cultivated endothelial cells.

As a consequence of these observations, a pharmaceutical composition suitable for administration to a patient suffering e.g. from ischemic or reperfusion damage of the heart is provided on the basis of the angiogenic effect of one or both of FGF9 and DKK1. Accordingly, a pharmaceutical composition comprising FGF9 and/or DKK1 is provided for administration in the treatment of heart damage resulting from ischemic and/or reperfusion conditions. Accordingly, the present invention provides the use of one or both of FGF9 and DKK1 for the production of a pharmaceutical composition for administration to a patient suffering heart damage, e.g. from an ischemic condition or reperfusion condition of the heart.

The angiogenic effect that is assumed to be caused by the therapy involving administration of autologous bone marrow cells in infarcted patients could be shown for FGF9 and/or DKK1 in a cell culture assay. In this assay, culture supernatants of bone marrow cells or FGF9 or DKK1, e.g. produced recombinantly in a micro-organism, were shown to have angiogenic effects on venous endothelial cells (HUVEC, human umbilical venous endothelial cells) and on cultivated human coronary arterial endothelial cells (HCAEC).

In an *in vitro* assay, neonatal ventricular rat cardiomyocytes could be protected from cell death by administration of culture supernatants of bone marrow cells or FGF9, e.g. produced recombinantly in a micro-organism, under the conditions of simulated ischemia, or under conditions of simulated ischemia in combination with subsequent reperfusion.

### Example 1: Analysis of FGF9 and DKK1 in infarcted patients

A detailed analysis of the bone marrow cell culture supernatant comparing the pattern of gene expression to the gene expression pattern of blood leukocytes of infarcted patients, a total of 26 factors could be identified that were expressed in bone marrow cells at least at a two-fold different level than in leukocytes. Using real-time PCR on mRNA isolated from cultivated bone marrow cells and from peripheral blood as well as analysis of proteins by ELISA, the following concentrations were obtained for FGF9 and DKK1, respectively, in infarcted patients receiving autologous bone marrow cell treatment :

**Table 1: Levels of FGF9 and DKK1 in cultivated bone marrow cells and peripheral blood**

| protein expression | sample | mean (pg/mL) | SD | SEM |
|---|---|---|---|---|
| FGF9 | bone marrow culture | 59.2 | 13.3 | 5.4 |
| FGF9 | blood | 34.4 | 9.1 | 3.7 |
| DKK1 | bone marrow culture | 55.9 | 22.5 | 9.2 |
| DKK1 | blood | 27.3 | 3.5 | 1.4 |
| mRNA | | | | |
| FGF9 | bone marrow culture | 7.3 | 4.1 | 1.4 |
| DKK1 | bone marrow culture | 3.4 | 2.3 | 0.8 |

### Example 2: Protective effect of FGF9 on cardiomyocytes against apoptosis and necrosis

Neonatal ventricular rat cardiomyocytes were subjected to a simulated ischemia for 3 hours in an *in vitro* assay and to subsequent reperfusion conditions for 1 hour. Setting the apoptosis rate to 100% for the rat cardiomyocytes as induced by ischemia / reperfusion (I/R), the addition of FGF9 in a range from 1 to 100 ng/mL could reduce I/R induced apoptosis by about 60%, as shown in the following table 3. For analysis of apoptosis, the TUNEL assay was used. In the experiments, cardioprotective growth differentiation factor 15 (GDF-15) was used as a positive control, and physiological saline as a negative control (control).

**Table 2: Effect of FGF9 in neonatal rat cardiomyocytes against apoptosis induced by ischemia / reperfusion**

| | | mean (%) | SD (%) | SEM (%) | p-value (compared to I/R) |
|---|---|---|---|---|---|
| control, no I/R | 0 ng/mL FGF9 | 21.3 | 7.7 | 3.8 | |
| I/R | 0 ng/mL FGF9 | 100 | 0 | 0 | |
| I/R | 1 ng/mL FGF9 | 96.0 | 23.6 | 8.3 | |
| I/R | 3 ng/mL FGF9 | 74.0 | 23.4 | 8.3 | |
| I/R | 10 ng/mL FGF9 | 47.4 | 23.9 | 8.4 | <0.05 |
| I/R | 30 ng/mL FGF9 | 54.4 | 12.8 | 4.5 | <0.05 |
| I/R | 100 ng/mL FGF9 | 59.8 | 7.1 | 2.5 | <0.05 |
| I/R | 20 ng/mL GDF-15 | 58.2 | 22.8 | 8.1 | <0.05 |

While ischemia / reperfusion conditions predominantly induce cell death by apoptosis, ischemia predominantly causes necrotic cell death in cardiomyocytes. It could be shown that FGF9 also exerts its protective effect on cardiomyocytes during simulated ischemia for 4 hours, reducing the rate of induced necrosis in neonatal ventricular rat cardiomyocytes by about 80%. This protective effect of FGF9 is comparable to that of GDF-15, as shown in table 3.

**Table 3: Protective effect of FGF9 against the necrosis used by simulated ischemia in neonatal ventricular rat cardiomyocytes**

| | | mean (%) | SD (%) | SEM (%) | p-value (compared to ischemia) |
|---|---|---|---|---|---|
| control, no I/R | 0 ng/mL FGF9 | 8.6 | 3.5 | 2.0 | |
| I/R | 0 ng/mL FGF9 | 100 | 0 | 0 | |
| I/R | 1 ng/mL FGF9 | 87.9 | 7.8 | 4.5 | |
| I/R | 3 ng/mL FGF9 | 62.3 | 7.3 | 4.2 | |
| I/R | 10 ng/mL FGF9 | 21.1 | 14.1 | 8.2 | <0.05 |
| I/R | 30 ng/mL FGF9 | 28.2 | 12.4 | 7.2 | <0.05 |
| I/R | 20 ng/mL GDF-15 | 32.4 | 9.8 | 5.7 | <0.05 |

Further, FGF9 could be shown to support in a dose dependent manner the migration of mononuclear cells, isolated from the peripheral blood using Percoll gradient centrifugation, as well as the migration of venous and arterial endothelial cells. Results are shown in table 4 and demonstrate the effect of FGF9 in stimulating migration of mononuclear cells. On the basis of the observation that mononuclear cells originating from bone marrow in addition to proliferating resident, terminally differentiated endothelial cells, participate in angiogenesis in ischemic tissue, migration of mononuclear cells from blood and the migration of venous and arterial endothelial cells is desirable to support angiogenesis in ischemic tissue. Accordingly, FGF9 can also be used for the preparation of a pharmaceutical composition for the induction of migration of cells, especially of bone marrow cells into ischemic tissue. As is shown below, DKK1 can used for the same purpose.

**Table 4: Induction of migration of mononuclear cells and endothelial cells by FGF9**

| | | mean (%) | SD (%) | SEM (%) | p-value (compared to control) |
|---|---|---|---|---|---|
| mononuclear cells | | | | | |
| control | 0 ng/mL | 100 | 0 | 0 | |
| FGF9 | 10 ng/mL | 125.1 | 70.9 | 35.4 | |
| FGF9 | 30 ng/mL | 233.3 | 126.8 | 63.4 | <0.05 |
| FGF9 | 100 ng/mL | 308.3 | 159.6 | 79.8 | <0.05 |
| | | | | | |

| HUVEC | | | | | |
|---|---|---|---|---|---|
| control | | 100 | 0 | 0 | |
| FGF9 | 3 ng/mL | 103.6 | 6.7 | 3.0 | |
| FGF9 | 10 ng/mL | 104.0 | 11.5 | 5.1 | |
| FGF9 | 30 ng/mL | 141.9 | 24.6 | 11.0 | <0.05 |
| FGF9 | 100 ng/mL | 159.9 | 38.2 | 17.1 | <0.05 |
| VEGF | 100 ng/mL | 265.2 | 70.5 | 31.5 | <0.05 |
| | | | | | |

| HCAEC | | | | | |
|---|---|---|---|---|---|
| control | | 100 | 0 | 0 | |
| FGF9 | 10 ng/mL | 102.5 | 5.3 | 3.1 | |
| FGF9 | 30 ng/mL | 126.3 | 8.8 | 5.1 | <0.05 |
| FGF9 | 100 ng/mL | 150.2 | 34.6 | 20.0 | <0.05 |
| VEGF | 100 ng/mL | 130.5 | 5.0 | 2.9 | <0.05 |

Further, it could be shown that FGF9 increases the proliferation of arterial endothelial cells, which is an important effect in angiogenesis, especially for the *de novo* generation of blood vessels in ischemic tissue by proliferation of resident endothelial cells. It could be shown in an *in vitro* assay that FGF9 caused a significant increase in the proliferation of coronary arterial endothelial cells to an extent comparable to the effect of VEGF. In contrast to coronary arterial endothelial cells, FGF9 did not influence proliferation of venous endothelial cells. Results are shown in table 5.

**Table 5: Induction of proliferation of arterial endothelial cells by FGF9**

| | | mean (%) | SD (%) | SEM (%) | p-value (compared to control) |
|---|---|---|---|---|---|
| HCAEC | | | | | |
| control | | 100 | 0 | 0 | |
| FGF9 | 1 ng/mL | 106.8 | 17.8 | 8.0 | |
| FGF9 | 3 ng/mL | 118.9 | 14.1 | 6.3 | |
| FGF9 | 10 ng/mL | 132.7 | 19.7 | 8.8 | <0.05 |
| FGF9 | 30 ng/mL | 138.9 | 23.6 | 10.6 | <0.05 |
| VEGF | 10 ng/mL | 129.6 | 13.9 | 8.0 | <0.05 |
| | | | | | |

| HUVEC | | | | | |
|---|---|---|---|---|---|
| control | | 100 | 0 | 0 | |
| FGF9 | 1 ng/mL | 101.8 | 14.1 | 6.3 | |
| FGF9 | 3 ng/mL | 110.3 | 21.2 | 9.5 | |
| FGF9 | 10 ng/mL | 104.5 | 17.6 | 7.9 | |
| FGF9 | 30 ng/mL | 106.1 | 20.6 | 9.2 | |
| VEGF | 10 ng/mL | 128.8 | 8.9 | 4.0 | <0.05 |

Further, FGF9 was shown to have a proangiogenic effect in venous and arterial endothelial cells in an *in vitro* assay. Known proangiogenic factors like VEGF induce the formation of tube-like structures in cultivated endothelial cells in the Matrigel assay according to Bodnar et al. (Circ. Res. 2006 March 17, 98 (5): 617 - 625). Another assay uses the sprouting of cells from murine aortic rings under *in vitro* cultivation for the determination of proangiogenic effects as e.g. described in Masson et al. (Biol. Proced. online 2002, 4: 24 - 31). It could be shown that FGF9 demonstrated a strong proangiogenic effect in both assays. In the aortic ring assay, the proangiogenic effect of FGF9 was found to an extent comparable to that of VEGF. Results are shown in table 6.

**Table 6: Endothelial cell tube formation and sprouting of endothelial cells from aortic rings**

| tube formation | | mean (%) | SD (%) | SEM (%) | p-value (compared to control) |
|---|---|---|---|---|---|
| HUVEC | | | | | |
| control | 0 ng/mL | 100 | 0 | 0 | |
| FGF9 | 1 ng/mL | 102.1 | 45.2 | 22.6 | |
| FGF9 | 3 ng/mL | 174.9 | 46.4 | 23.2 | <0.05 |
| FGF9 | 10 ng/mL | 219.7 | 51.8 | 25.9 | <0.05 |
| FGF9 | 30 ng/mL | 201.3 | 50.7 | 25.4 | <0.05 |
| VEGF | 10 ng/mL | 379.0 | 94.4 | 47.2 | <0.05 |
| | | | | | |

| HCAEC | | | | | |
|---|---|---|---|---|---|
| control | | 100 | 0 | 0 | |
| FGF9 | 1 ng/mL | 192.6 | 24.7 | 14.3 | <0.05 |
| FGF9 | 3 ng/mL | 270.2 | 7.2 | 4.1 | <0.05 |
| FGF9 | 10 ng/mL | 322.3 | 19.9 | 11.5 | <0.05 |
| VEGF | 10 ng/mL | 227.0 | 42.8 | 24.7 | <0.05 |
| | | | | | |

| murine aortic ring assay | | µm | µm | µm | |
|---|---|---|---|---|---|
| day 3 | control | 11.2 | 14.3 | 6.4 | |
| day 3 | 100 ng/mL FGF9 | 234.1 | 27.4 | 13.7 | <0.05 |
| day 4 | control | 184.5 | 11.8 | 5.3 | |
| day 4 | 100 ng/mL FGF9 | 486.7 | 67.2 | 33.6 | <0.05 |
| day 5 | control | 238.7 | 33.3 | 16.7 | |
| day 5 | 100 ng/mL FGF9 | 517.2 | 143.0 | 71.5 | <0.05 |
| day 6 | control | 257.96 | 49.8 | 24.9 | |
| day 6 | 100 ng/mL FGF9 | 834.65 | 133.1 | 66.6 | <0.05 |
| day 13 | control | 277.5 | 75.3 | 37.6 | |
| day 13 | 100 ng/mL FGF9 | 1137.4 | 106.5 | 53.3 | <0.05 |

### Example 3: Cardioprotective effect of FGF9 in acute myocardial infarction in vivo

Using a rat with a coronary ligature for generating an acute myocardial infarction as an animal model, it could be shown that FGF9 significantly reduces size and extent of the infarction and causing an improved function of the left ventricle, which was damaged by the infarction. In detail, FGF9 (4 µg) was administered by intramyocardial injection immediately after placement of a permanent coronary ligature in a rat under approved animal testing conditions.

It could be shown that FGF9 resulted in a significant reduction of the size and extent of the infarction by TTC staining (as described in Parsa et al., J. Clin. Invest. 112, 999-1007 (2003)) in tissue sections prepared 24 h after placement of the ligature in comparison to a parallel experiment without injection of FGF9. Further, echocardiographic analysis (apparatus by VisualSonics) in the experimental animals 24 h after placement of the ligature with and without injection of FGF9 showed an improved function of the left ventricle that was subject to the infarction. Results are shown in table 7.

**Table 7: Injection of FGF9 protein following an acute myocardial infarction reduces the extent of infarction and reduces impairment of ventricle function**

| | mean (%) | SD (%) | SEM (%) | p-value (compared to I control) |
|---|---|---|---|---|
| size of infarction in % of entire left ventricle | | | | |
| control | 38.7 | 2.4 | 6.5 | |
| FGF9 | 27.8 | 7.7 | 2.9 | <0.05 |

| echocardiographic analysis (fractional area change) | | | | |
|---|---|---|---|---|
| control | 18.8 | 5.6 | 2.1 | |
| FGF9 | 28.5 | 4.1 | 1.5 | <0.05 |

The above data demonstrate that FGF9 is a pleiotropic growth factor having a strong proangiogenic effect on endothelial cells, FGF9 induces migration of endothelial cells and monocytes in combination with a strong cardioprotective effect on cardiomyocytes following infarction, and especially, FGF9 has a strong anti-apoptotic and anti-necrotic effect against damage caused by ischemia / reperfusion and ischemia.

### Example 4: Angiogenic effect of DKK1

Using the assay described in the above examples with DKK1 instead of FGF9, it could be shown that DKK1 in a dose dependent manner stimulates migration of venous endothelial cells. Stimulation of migration by DKK1 was comparable to the effect of VEGF. Results are shown in table 8.

**Table 8: Induction of migration of venous endothelial cells by DKK1**

| | | mean (%) | SD (%) | SEM (%) | p-value (compared to control) |
|---|---|---|---|---|---|
| HUVEC | | | | | |
| control | | 100 | 0 | 0 | |
| DKK1 | 3 ng/mL | 127.4 | 30.2 | 13.5 | |
| DKK1 | 10 ng/mL | 214.8 | 93.6 | 41.9 | |
| DKK1 | 30 ng/mL | 292.4 | 92.8 | 41.5 | <0.05 |
| DKK1 | 100 ng/mL | 304.5 | 51.1 | 22.9 | <0.05 |
| VEGF | 100 ng/mL | 265.2 | 70.5 | 31.5 | <0.05 |

These results demonstrate that DKK1 has an angiogenic effect by inducing migration of venous endothelial cells.

Further, DKK1 could be shown to have proangiogenic effects on venous and arterial endothelial cells. Using an in vitro assay of cultivated human umbilical venous endothelial cells or, alternatively, the aortic ring assay as described in Example 3, DKK1 was demonstrated to cause the formation of tubular structures in the venous endothelial cells and induces the sprouting of cells from aortic rings. Results are shown in table 9.

**Table 9: Endothelial cell tube formation and sprouting of endothelial cells from aortic rings**

| tube formation | | mean (%) | SD (%) | SEM (%) | p-value (compared to control) |
|---|---|---|---|---|---|
| HUVEC | | | | | |
| control | 0 ng/mL | 100 | 0 | 0 | |
| DKK1 | 0.33 ng/mL | 110.5 | 51.8 | 25.9 | |
| DKK1 | 3 ng/mL | 268.4 | 58.6 | 29.3 | <0.05 |
| DKK1 | 10 ng/mL | 389.8 | 37.4 | 18.7 | <0.05 |
| DKK1 | 30 ng/mL | 359.0 | 92.8 | 46.6 | <0.05 |
| VEGF | 10 ng/mL | 379.0 | 94.4 | 47.2 | <0.05 |

| | | mean (%) | SD (%) | SEM (%) | p-value (compared to control) |
|---|---|---|---|---|---|
| murine aortic ring assay | | µm | µm | µm | |
| day 3 | control | 111.2 | 14.3 | 6.4 | |
| day 3 | 100 ng/mL DKK1 | 190.1 | 48.2 | 21.6 | <0.05 |
| day 4 | control | 184.5 | 11.8 | 5.28 | |
| day 4 | 100 ng/mL DKK1 | 296.7 | 49.2 | 22.0 | <0.05 |
| day 5 | control | 238.7 | 33.3 | 16.7 | |
| day 5 | 100 ng/mL DKK1 | 405.8 | 84.6 | 37.8 | <0.05 |
| day 6 | control | 257.9 | 49.8 | 24.9 | |
| day 6 | 100 ng/mL DKK1 | 581.3 | 41.6 | 18.6 | <0.05 |
| day 13 | control | 277.5 | 75.3 | 37.6 | |
| day 13 | 100 ng/mL DKK1 | 630.9 | 71.6 | 32.0 | <0.05 |

Both FGF9 and DKK1 could be shown to have strong proangiogenic effects, making these proteins active pharmaceutically ingredients for use in the treatment of tissue damage caused e.g. by ischemic and/or reperfusion conditions of the heart.

## Claims

1. Use of FGF9 and/or of DKK1 for the production of a pharmaceutical composition for the treatment of myocardium damage inducible by ischemic and/or reperfusion conditions.

2. Use according to claim 1, **characterized in that** the ischemic and/or reperfusion conditions are selected from the group comprising myocardial infarction, angina, vessel occlusion, peripheral arterial obstructive disease, congestive heart failure and chronic heart failure.

3. Use according to one of the preceding claims, **characterized in that** FGF9 has an amino acid sequence according to Seq ID No. 1 and DKK1 has an amino acid sequence according to Seq ID No. 2.

4. Use according to one of claims 1 to 2, **characterized in that** FGF9 is a functional derivative or a functional mutant of the Seq ID No. 1 essentially having the angiogenic and/or cytoprotective effect of Seq ID No. 1.

5. Use according to one of claims 1 to 2, **characterized in that** DKK1 is a functional derivative or a functional mutant of the Seq ID No. 2 essentially having the angiogenic effect of Seq ID No. 2.

6. Use according to one of the preceding claims, **characterized in that** FGF9 and/or DKK1 is provided as a nucleic acid sequence encoding FGF9, DKK1 and/or a functional derivative or mutant nucleic acid sequence thereof.

7. Use according to one of the preceding claims, **characterized** that FGF9 and/or DKK1 is administered for inducing angiogenesis.

8. Use according to one of the preceding claims, **characterized in that** FGF9 is administered for protecting cardiomyocytes from damage inducible by ischemic and/or reperfusion conditions.

9. Pharmaceutical composition for the treatment of myocardium damage inducible by ischemic and/or reperfusion conditions, **characterized by** comprising FGF9 and/or of DKK1.

10. Pharmaceutical composition according to claim 8, **characterized in that** the ischemic and/or reperfusion conditions are one ore more selected from the group comprising myocardial infarction, angina, vessel occlusion, peripheral arterial obstructive disease, congestive heart failure and chronic heart failure.

11. Pharmaceutical composition according to claim 8 or 9, **characterized in that** FGF9 has an amino acid sequence according to Seq ID No. 1 and DKK1 has an amino acid sequence according to Seq ID No. 2.

12. Pharmaceutical composition according to claim 8 or 9, **characterized in that** FGF9 is present in the form of a nucleic acid encoding an amino acid sequence according to Seq ID No. 1, and DKK1 is present in the form of a nucleic acid encoding an amino acid sequence according to Seq ID No. 2.

13. Pharmaceutical composition according to one of claims 8 to 12, **characterized in that** FGF9 is a functional derivative or a functional mutant of the Seq ID No. 1 essentially having the angiogenic and/or cytoprotective effect of Seq ID No. 1.

14. Pharmaceutical composition according to one of claims 8 to 12, **characterized in that** DKK1 is a functional derivative or a functional mutant of the Seq ID No. 2 essentially having the angiogenic effect of Seq ID No. 2.


SEQUENCE LISTING

<110>  Medizinische Hochschule Hannover

<120>  Pharmaceutical composition for the treatment of myocardial
       infarction

<130>  M1006EP

<160>  2

<170>  PatentIn version 3.4

<210>  1
<211>  208
<212>  PRT
<213>  Homo sapiens

<400>  1

<210>  2
<211>  266
<212>  PRT
<213>  Homo sapiens

<400>  2

Met Met Ala Leu Gly Ala Ala Gly Ala Thr Arg Val Phe Val Ala Met
1               5                   10                  15

Val Ala Ala Ala Leu Gly Gly His Pro Leu Leu Gly Val Ser Ala Thr
            20                  25                  30

Leu Asn Ser Val Leu Asn Ser Asn Ala Ile Lys Asn Leu Pro Pro Pro
        35                  40                  45

Leu Gly Gly Ala Ala Gly His Pro Gly Ser Ala Val Ser Ala Ala Pro
    50                  55                  60

Gly Ile Leu Tyr Pro Gly Gly Asn Lys Tyr Gln Thr Ile Asp Asn Tyr
65                  70                  75                  80

Gln Pro Tyr Pro Cys Ala Glu Asp Glu Glu Cys Gly Thr Asp Glu Tyr
                85                  90                  95

Cys Ala Ser Pro Thr Arg Gly Gly Asp Ala Gly Val Gln Ile Cys Leu
            100                 105                 110

Ala Cys Arg Lys Arg Arg Lys Arg Cys Met Arg His Ala Met Cys Cys
        115                 120                 125

Pro Gly Asn Tyr Cys Lys Asn Gly Ile Cys Val Ser Ser Asp Gln Asn
    130                 135                 140

His Phe Arg Gly Glu Ile Glu Glu Thr Ile Thr Glu Ser Phe Gly Asn
145                 150                 155                 160

Asp His Ser Thr Leu Asp Gly Tyr Ser Arg Arg Thr Thr Leu Ser Ser
                165                 170                 175

Lys Met Tyr His Thr Lys Gly Gln Glu Gly Ser Val Cys Leu Arg Ser
            180                 185                 190

Ser Asp Cys Ala Ser Gly Leu Cys Cys Ala Arg His Phe Trp Ser Lys
        195                 200                 205

Ile Cys Lys Pro Val Leu Lys Glu Gly Gln Val Cys Thr Lys His Arg
    210                 215                 220

Arg Lys Gly Ser His Gly Leu Glu Ile Phe Gln Arg Cys Tyr Cys Gly
225                 230                 235                 240

Glu Gly Leu Ser Cys Arg Ile Gln Lys Asp His His Gln Ala Ser Asn
                245                 250                 255

Ser Ser Arg Leu His Thr Cys Gln Arg His
            260                 265